Europäisches Patentamt

European Patent Office

Office européen des brevets

(19)

(11) Numéro de publication: **0 335 807**
**A1**

(12) **DEMANDE DE BREVET EUROPEEN**

(21) Numéro de dépôt: **89420107.8**

(22) Date de dépôt: **24.03.89**

(51) Int. Cl.⁴: **F 16 K 11/074**
**A 61 M 5/16**

(30) Priorité: **28.03.88 FR 8804554**

(43) Date de publication de la demande:
**04.10.89 Bulletin 89/40**

(84) Etats contractants désignés:
**AT BE CH DE ES FR GB GR IT LI LU NL SE**

(71) Demandeur: **CAIR (SOCIETE ANONYME DE DROIT FRANCAIS)**
**4-6, rue de l'Union**
**F-69170 Tarare (FR)**

(72) Inventeur: **Lopez, Georges Antoine**
**Rue des Phily**
**F-69290 Craponne (FR)**

**Bouvier, Bernard**
**57 rue H. Richaume Escalier B4**
**F-78360 Montesson (FR)**

(74) Mandataire: **Maureau, Philippe et al**
**Cabinet GERMAIN & MAUREAU BP 3011**
**F-69392 Lyon Cédex 03 (FR)**

(54) **Robinet à plusieurs voies pour usage médical.**

(57) Ce robinet comprend deux flasques parallèles (5,6) dans lesquels débouchent deux tubulures coaxiales (10,12) et entre lesquels est montée pivotante, autour d'un axe (13) perpendiculaire aux flasques, une pièce formant boisseau (7) d'épaisseur correspondant à l'écartement entre les flasques, dans laquelle sont ménagés, à une distance de l'axe de rotation (13) correspondant à la distance entre l'axe de rotation du boisseau et l'axe des tubulures débouchant dans les flasques, deux perçages (14, 15) décalés angulairement l'un par rapport à l'autre dans l'un (15) desquels débouche radialement, directement ou non une tubulure de connexion (16), des moyens (19,20,22) étant destinés à assurer l'étanchéité entre le boisseau (7) de chaque flasque (5,6) au niveau des passages de liquide.
Application à l'équipement de dispositifs de perfusion.

FIG.2

# Description

## Robinet à plusieurs voies pour usage médical

La présente invention a pour objet un robinet à plusieurs voies pour usage médical.

Il est fréquent, dans les services de médecine, de chirurgie ou de réanimation d'avoir à perfuser plusieurs liquides simultanément.

A cet effet, sont utilisés des sites à connexions multiples permettant la sélection de voies préférentielles de passage de différents liquides. Les dispositifs connus se présentent sous la forme d'un robinet ou d'une rampe de robinets à trois ou quatre voies, selon le nombre de liquides à perfuser.

Chaque robinet ou rampe de robinets comprend un corps moulé en matière synthétique, telle qu'en polycarbonate ou polyamide, équipé de l'ensemble de la tuyauterie nécessaire au passage des fluides, et des connexions normalisées pour le montage sur des tubulures reliées aux contenants des drogues à administrer (flacons, poches, pousse-seringues, seringues, etc.) ainsi qu'à la tubulure reliée au patient par l'intermédiaire d'un cathéter.

Chaque robinet comporte également un boisseau, généralement en polyéthylène, possédant un système de sélection des voies et actionnable par un maneton. Le montage du boisseau à l'intérieur du corps du robinet est effectué par clipsage, la matière du boisseau, moins rigide que celle du corps, lui permettant de rentrer en force par pression.

Ces systèmes sont généralement transparents, afin de faciliter la visualisation des différents flux de liquides susceptibles de circuler. Avec un robinet traditionnel, il est possible de réaliser plusieurs fonctions :
- écoulement dans le canal principal,
- écoulement dans le canal principal et dans un canal latéral,
- écoulement dans un canal latéral seul avec fermeture du canal principal, notamment lors des prises de pression veineuse centrale au niveau de l'oreillette droite,
- fermeture totale du robinet.

Ces sites de connexion sont appelés à être manoeuvres plusieurs fois par jour tant au niveau des manettes de manoeuvre qu'au niveau des connexions proprement dites.

Ces manipulations multiples sont à la source de fautes d'asepsie conduisant à une contamination du système par introduction de germes dans ceux-ci puis dans l'organisme du patient.

Pour remédier à ces inconvénients, il a été imaginé d'enrober les sites de connexion dans des pansements imbibés d'agents antiseptiques. Une telle solution n'est pas satisfaisante car nécessitant, pour chaque manipulation du site, un retrait du pansement, avant nouvelle remise en place de celui-ci. Or, un tel pansement est salissant tant pour le patient que pour le personnel médical.

Dans ces conditions, l'enrobage par un pansement a été remplacé par un enrobage par un boîtier en matière synthétique contenant une mousse imbibée d'un agent antiseptique en contact avec la totalité des zones de connexion et de manipulation.

Toutefois, ces dispositifs étant monoblocs et totalement opaques, éliminent la possibilité de visualiser la position des manettes ainsi que le flux dans le canal principal.

En outre, toute manipulation d'un boisseau est réalisée après ouverture du boîtier, les doigts de l'opérateur venant en contact avec l'antiseptique qui se trouve au contact de la mousse.

Enfin, les défauts éventuels d'étanchéité ne peuvent en aucun cas être visualisés à l'intérieur du boîtier, empêchant toute intervention rapide du personnel médical.

Le but de la présente invention est de fournir un robinet à usage médical de structure simple, fonctionnant dans d'excellentes conditions d'étanchéité, pouvant être maintenu en conditions stériles en période d'utilisation tout en permettant au personnel médical de visualiser immédiatement le sens de circulation des fluides, et d'actionner le robinet sans avoir à ouvrir un quelconque boîtier de protection ni à venir en contact avec le liquide antiseptique.

A cet effet, le robinet qu'elle concerne comprend deux flasques parallèles dans lesquels débouchent deux tubulures coaxiales et entre lesquels est montée pivotante, autour d'un axe perpendiculaire aux flasques, une pièce formant boisseau d'épaisseur correspondant à l'écartement entre les flasques, dans laquelle sont ménagés, à une distance de l'axe de rotation correspondant à la distance entre l'axe de rotation du boisseau et l'axe des tubulures débouchant dans les flasques, deux perçages décalés angulairement l'un par rapport à l'autre dans l'un desquels débouche radialement, directement ou non une tubulure de connexion, des moyens étant destinés à assurer l'étanchéité entre le boisseau de chaque flasque au niveau des passages de liquide.

Avantageusement, les deux flasques et le boisseau qui leur est associé sont de forme circulaire, l'axe de rotation du boisseau correspondant à l'axe des flasques.

Afin de faciliter la manipulation du robinet, et notamment de fournir un positionnement précis du boisseau dans chaque position, ce dernier comporte, à sa périphérie plusieurs évidements décalés angulairement les uns par rapport aux autres des mêmes valeurs que les différentes positions de fonctionnement du boisseau, un doigt monté dans le support du robinet et poussé élastiquement par un ressort étant destiné à pénétrer, pour chaque position du boisseau, dans un des évidements que comporte celui-ci. Ce doigt pourrait également venir de moulage avec le support et posséder une forme lui donnant une élasticité suffisante.

Selon une forme d'exécution de ce robinet, les moyens assurant l'étanchéité entre le boisseau et chaque flasque sont constitués par un système de joints solidaires du flasque ou du boisseau et engagés dans des rainures ménagées dans l'une ou l'autre de ces pièces, comprenant un joint annulaire entourant l'axe de rotation, un joint annulaire disposé à proximité de la périphérie des pièces et des joints entourant les perçages ménagés axialement dans le boisseau.

Il est à noter que chaque système de joint peut venir de moulage en une seule piece.

Avantageusement, ce robinet comporte, ménagée dans un flasque ou dans la face correspondante du boisseau, une rainure circulaire centrée sur l'axe de rotation du boisseau et située à même distance de l'axe que les perçages du boisseau, s'étendant à partir de l'un des perçages du flasque ou du boisseau et susceptible de mettre en communication la tubulure débouchant dans l'un des flasques avec le perçage du boisseau dans lequel débouche une tubulure radiale.

Cette rainure est mise à profit pour faire communiquer une tubulure du canal principal avec la tubulure latérale.

Conformément à une autre caractéristique de l'invention, le flasque situé du côté opposé à celui présentant une rainure de mise en communication d'une tubulure axiale et d'une tubulure radiale comporte une rainure circulaire centrée sur l'axe de rotation du boisseau et située à même distance de l'axe que les perçages du boisseau, s'étendant à partir de la tubulure du flasque dans lequel elle est ménagée, sur un angle correspondant au décalage angulaire des deux perçages ménagés dans le boisseau. Cette disposition permet de réaliser la stérilisation de tous les compartiments de l'ensemble hydraulique et de réaliser la purge des canaux lors de la mise en service du système.

Avantageusement, lorsque ce robinet est destiné à être utilisé dans d'excellentes conditions d'asepsie, il est monté à l'intérieur d'un boîtier comprenant une partie rigide contenant de la mousse susceptible d'être imbibée d'agent antiseptique, le boîtier comprenant une fente disposée dans le plan du boisseau, destinée à laisser le passage des moyens d'actionnement de celui-ci. Dans ce cas, les moyens d'actionnement du boisseau sont constitués par un levier radial, de longueur supérieure à la dimension radiale maximale du boîtier, considérée à partir de l'axe de rotation.

Le levier d'actionnement du boisseau faisant en permanence saillie du boîtier indique immédiatement à l'opérateur quelle est la situation dans laquelle se trouve le robinet, l'opérateur pouvant actionner le levier et par suite changer le robinet d'état depuis l'extérieur, c'est-à-dire sans aucune modification des conditions d'asepsie.

Conformément à une autre caractéristique de l'invention, le boîtier présente une dissymétrie de révolution, certaines zones se trouvant à une distance de l'axe de pivotement inférieure à la distance entre la connexion de la tubulure radiale et l'axe de rotation. Cela permet, en fonction de la position du levier, soit de dégager la connexion radiale hors du boîtier pour la relier à une tubulure, soit au contraire de la maintenir à l'intérieur de la mousse pour réaliser sa protection.

Selon une autre forme d'exécution de ce robinet, les moyens d'actionnement du boisseau sont constitués par un levier radial solidaire de celui-ci et sortant du boîtier, sur lequel sont montés, de façon pivotante élastiquement, deux bras qui en sont parallèles et qui sont disposés de part et d'autre de lui, une des extrémités libre de chacun de ces deux bras étant au contact d'une rampe fixe crantée, lorsqu'aucune action n'est exercée sur ceux-ci, les crans de chaque rampe correspondant aux différentes positions de fonctionnement du boisseau.

Pour permettre le pivotement du boisseau d'une position à une autre, il convient d'exercer une pression dans un sens de rapprochement des extrémités des bras opposées à celles en prise avec les rampes crantées. Ces dernières extrémités se dégagent des rampes, permettant un libre pivotement du boisseau, lorsque celui-ci est dans la position souhaitée, les bras sont relâchés et viennent se verrouiller dans les crans des rampes.

De toute façon, l'invention sera bien comprise à l'aide de la description qui suit, en référence au dessin schématique annexé représentant, à titre d'exemple non limitatif, une forme d'exécution d'une rampe de robinets :

Figure 1 est une vue en perspective d'une rampe de trois robinets ;

Figure 2 est une vue en perspective éclatée des pièces essentielles constitutives d'un robinet, vues sous un premier angle ;

Figure 3 est une vue en perspective des pièces de figure 2 vues sous un autre angle ;

Figure 4 est une vue en coupe du boîtier selon la ligne IV-IV de figure 1 ;

Figure 5 est une vue en coupe du robinet selon la ligne V-V de figure 1 ;

Figures 6 à 9 sont quatre vues correspondant à quatre modes de fonctionnement de ce robinet, chaque figure représentant, vus de face, les deux flasques et le boisseau ;

Figures 10 et 11 sont deux vues de face et de côté, d'une autre rampe à deux robinets.

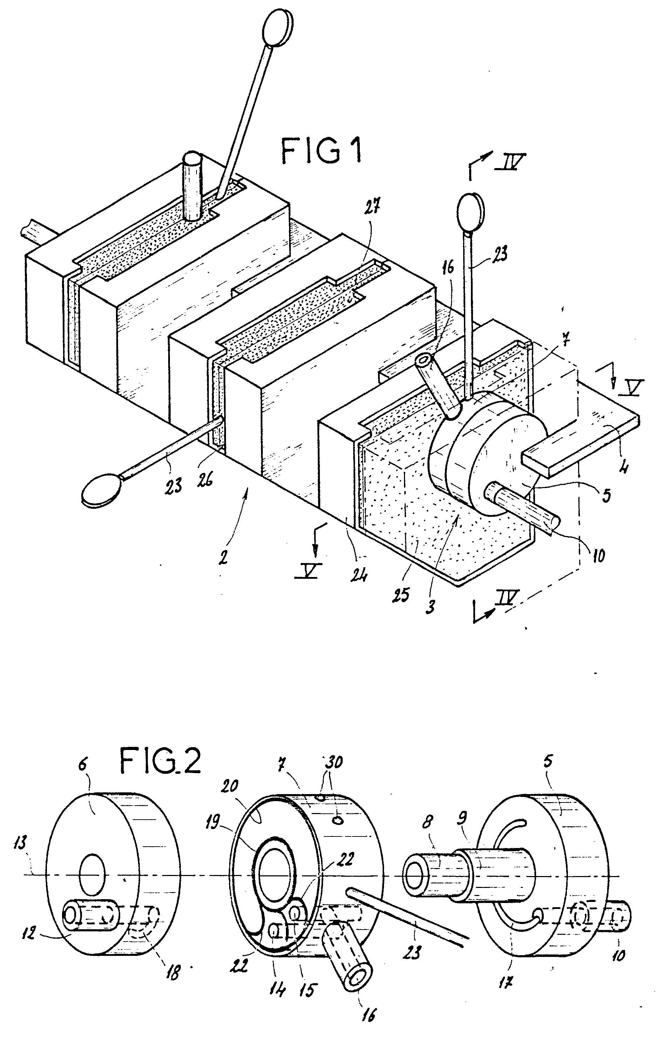

La figure 1 représente une rampe de robinets 2 comprenant trois robinets 3 identiques, montés sur un support commun 4. Chaque robinet 3 comprend deux flasques circulaires 5 et 6 coaxiaux, entre lesquels est monté un boisseau 7 également circulaire, coaxial aux flasques 5 et 6, et d'épaisseur correspondant à la distance entre les deux flasques 5 et 6.

Le flasque 5 comporte un doigt axial 8 sur lequel sont montés, d'une part, le boisseau 7 avec interposition d'un manchon 9 facilitant son pivotement et, d'autre part, le flasque 6 qui est immobilisé sur le doigt 8.

Dans chacun des flasques 5 et 6, débouchent deux tubulures 10 et 12 constitutives du canal principal d'écoulement de fluides. Les tubulures 10 et 12 sont coaxiales et orientées parallèlement à l'axe 13 de rotation du boisseau. A l'intérieur du boisseau 7 sont ménagés deux perçages 14 et 15 parallèles à l'axe 13, situés à même distance de celui-ci que les tubulures 10 et 12, et décalés angulairement l'un par rapport à l'autre. A l'intérieur du perçage 15 débouche radialement une tubulure 16 destinée à réaliser une connexion latérale.

Comme montré à la figure 2, dans la face du flasque 5 située en regard du boisseau 7, est ménagée une rainure 17 débouchant dans la tubulure 10, s'étendant sur une portion de cercle, en vue d'une mise en communication de la tubulure 10 avec le perçage 15 pour une position déterminée du boisseau.

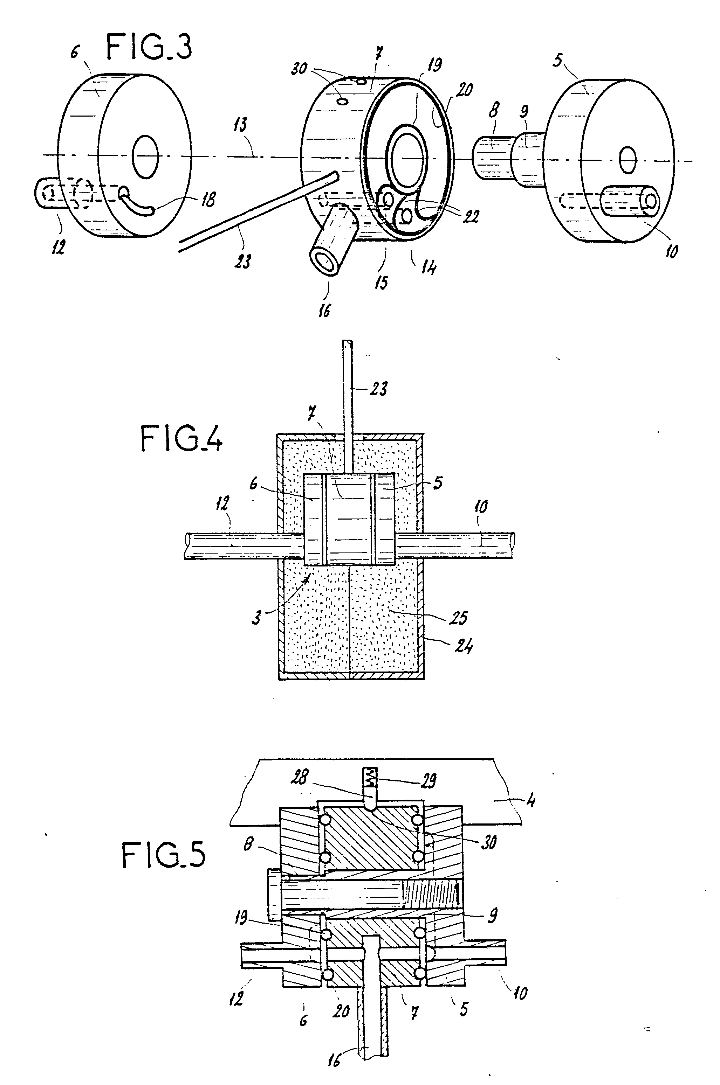

Comme montré à la figure 3, dans la face du flasque 6 tournée vers le boisseau, est ménagée une rainure 18 débouchant dans la tubulure 12 et s'étendant sur une ligne circulaire sur une longueur au moins égale au décalage angulaire entre les deux perçages 14 et 15 du boisseau.

L'étanchéité entre le boisseau 7 et chaque flasque 5,6 est réalisée par un système de joints, monté sur le boisseau, dans la forme d'exécution représentée au dessin, comprenant une partie 19 entourant l'axe de rotation du boisseau, une partie 20 disposée à proximité de la périphérie du boisseau, ainsi que des parties 22 reliant les parties 19 et 20 situées entre celles-ci ainsi que de part et d'autre de celles-ci.

L'actionnement du boisseau est réalisé par l'intermédiaire d'un levier radial 23 solidaire de celui-ci.

Comme montré notamment aux figures 1 et 3, chaque robinet est monté à l'intérieur d'un boîtier 24 en matière synthétique rigide dont l'intérieur est tapissé d'une mousse 25, telle qu'un polyuréthane, susceptible d'être imbibée d'agents antiseptiques. Ce boîtier 24 ainsi que la mousse 25 comprennent des fentes respectivement 26,27, permettant le passage du levier 23.

Comme cela ressort de la figure 1, le boîtier 24 ne présente pas de symétrie de révolution afin de permettre, pour une position du levier correspondant à celle du robinet intermédiaire de figure 1, l'introduction totale de la tubulure de connexion 16 à l'intérieur de la mousse et inversement, pour d'autres positions du boisseau, la sortie de cette tubulure hors de la mousse pour réaliser sa liaison avec une autre ligne.

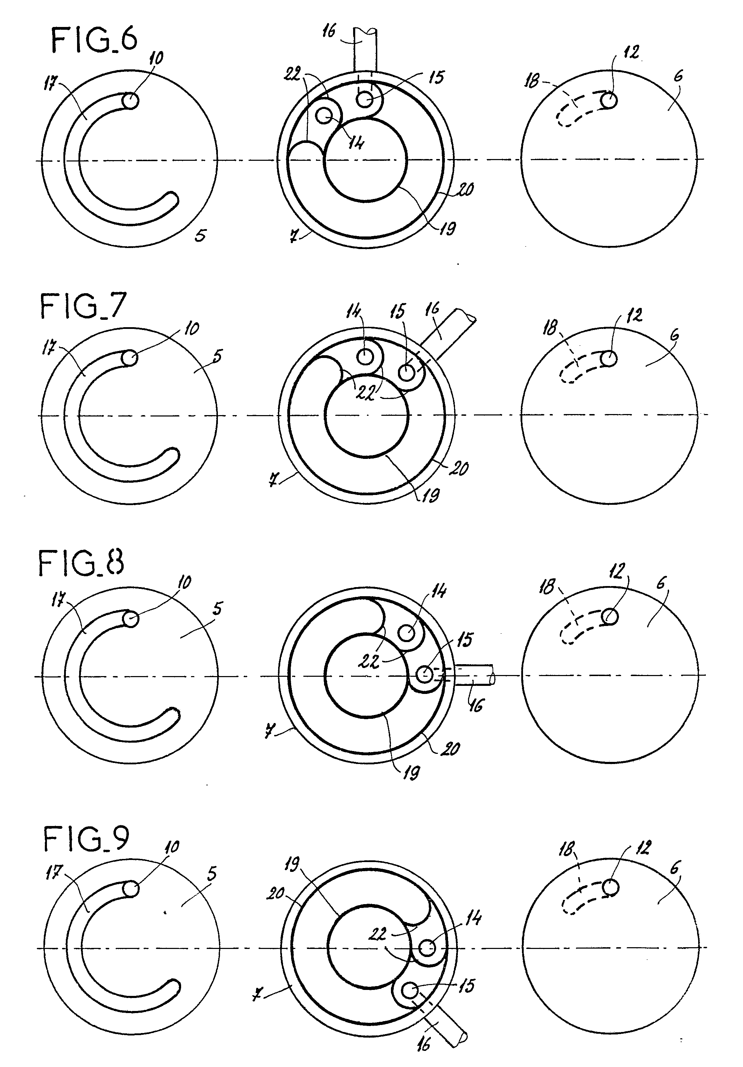

Comme cela ressort de la figure 5, le support 4 de chaque robinet comporte un doigt 28 faisant saillie radialement en direction du boisseau, maintenu poussé contre celui-ci par un ressort 29 et destiné à pénétrer dans des évidements 30 débouchant à la périphérie du boisseau, afin de réaliser l'immobilisation de celui-ci dans chaque position du robinet.

Chaque robinet de la rampe peut occuper les positions suivantes. Dans la position représentée à la figure 6, les tubulures 10 et 12 sont en regard du perçage 15 du boisseau. Cette position permet de mélanger un liquide provenant de la tubulure 16 à un liquide qui s'écoule dans le canal principal formé par les tubulures 10,12.

Cette position permet la stérilisation de tous les compartiments internes de l'ensemble hydraulique, les rainures 17 et 18 permettant au gaz de stérilisation d'aller dans les différents compartiments limités par les joints 19, 20, 22.

Cette position permet également la purge des tubulures 10, 12, 15 et 14 lors de la mise en service du système, les rainures 17 et 18 permettant la purge du perçage 14.

Dans la position représentée à la figure 7, le perçage 14 du boisseau se trouve en regard des tubulures 10 et 12, assurant un écoulement direct du liquide dans le canal principal sans être mélangé avec un liquide en provenance d'un autre canal.

La figure 8 correspond à une position de fermeture totale du robinet dans laquelle les tubulures 10, 12, 16 ne peuvent être mises en relation ni directement, ni par les rainures 17, 18.

Dans la position représentée à la figure 9, la tubulure 10 est mise en communication avec la tubulure 16 par l'intermédiaire de la rainure 17. Cette position permet à un liquide de s'écouler de la tubulure 10 dans la tubulure 16 et permet également la purge du canal 16 avant connexion.

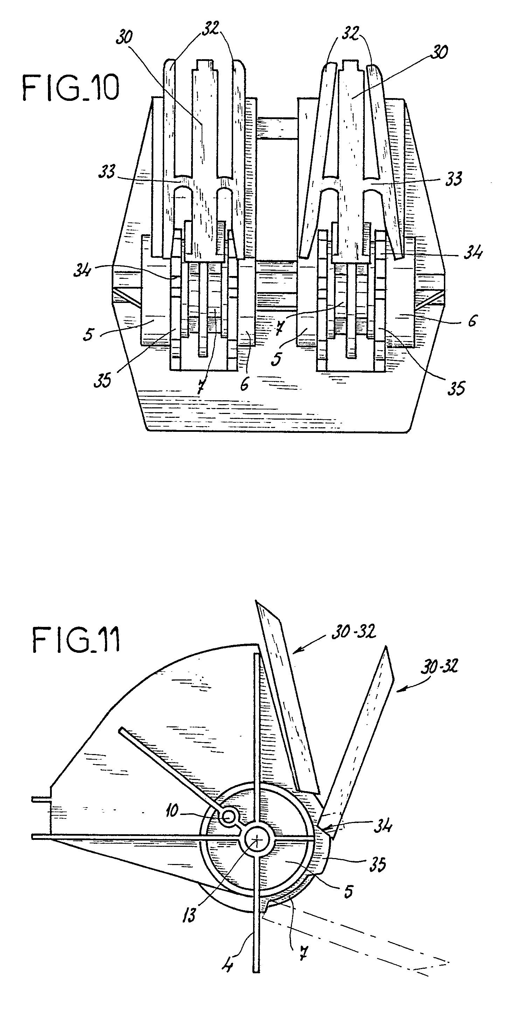

Les figures 10 et 11 représentent une variante d'exécution de ce robinet, dans lequel les mêmes éléments sont désignés par les mêmes références que précédemment. Dans ce cas, les moyens d'actionnement de chaque boisseau 7 sont constitués par un levier 30 en matière synthétique avec lequel viennent de moulage deux bras 32 qui en sont parallèles et qui lui sont reliés par deux pontets 33 disposés sensiblement à mi-longueur. Une extrémité de chacun des deux bras 32 est destinée, en position de repos des bras, à être engagée dans des crans 34 ménagés dans des rampes 35 solidaires du corps. Les crans 34 sont disposés pour correspondre aux différentes positions de fonctionnement du robinet. Un appui simultané, dans un sens de rapprochement, sur les deux extrémités libres des bras 32 permet, comme montré à la partie droite de la figure 10, de dégager leurs autres extrémités des crans que comportent les rampes, ce qui rend possible le pivotement du levier 30.

Comme il ressort de ce qui précède, l'invention apporte une grande amélioration à la technique existante en fournissant un robinet multi-voies à usage médical de conception simple, travaillant dans d'excellentes conditions d'étanchéité et susceptible d'être monté à l'intérieur d'un boîtier de connexion tout en pouvant être actionné ssans ouverture de ce boîtier et tout en permettant la visualisation instantanée de la position du boisseau.

Comme il va de soi, l'invention ne se limite pas à la forme d'exécution de ce robinet décrite ci-dessus à titre d'exemple ; elle en embrasse, au contraire, toutes les variantes.

C'est ainsi, notamment, que ce robinet pourrait comprendre un nombre différent de voies, que les rainures 17, 18, pourraient être ménagées dans le boisseau, que les joints pourraient être montés dans les flasques ou que les moyens d'actionnement en rotation du boisseau pourraient être différents, que la tubulure radiale 16 pourrait être envisagée dans l'un des flasques, que le boisseau pourrait être en forme de secteur circulaire, ou encore que le boîtier pourrait venir de moulage avec les flasques, sans que l'on sorte pour autant du cadre de l'invention.

## Revendications

1.- Robinet à plusieurs voies pour usage médical, caractérisé en ce qu'il comprend deux flasques parallèles (5,6) dans lesquels débouchent deux tubulures coaxiales (10,12) et entre lesquels est montée pivotante, autour d'un axe (13) perpendiculaire aux flasques, une pièce formant boisseau (7) d'épaisseur correspondant à l'écartement entre les flasques, dans laquelle sont ménagés, à une distance de l'axe de rotation (13) correspondant à la distance entre l'axe de rotation du boisseau et l'axe des tubulures débouchant dans les flasques, deux perçages (14,15) décalés angulairement l'un par rapport à l'autre dans l'un (15) desquels débouche radialement, directement ou non une tubulure de connexion (16), des moyens (19,20,22) étant destinés à assurer l'étanchéité entre le boisseau (7) et chaque flasque (5,6) au niveau des passages de liquide.

2.- Robinet selon la revendication 1, caractérisé en ce que les deux flasques (5,6) et le boisseau (7) qui leur est associé sont de forme circulaire, l'axe de rotation (13) du boisseau (7) correspondant à l'axe des flasques.

3.- Robinet selon la revendication 2, caractérisé en ce que le boisseau comporte, à sa périphérie, plusieurs évidements (30) décalés angulairement les uns par rapport aux autres des mêmes valeurs que les différentes positions de fonctionnement du boisseau, un doigt (28) monté dans le support (4) du robinet et poussé élastiquement par un ressort (29) étant destiné à pénétrer, pour chaque position du boisseau, dans un des évidements que comporte celui-ci.

4.- Robinet selon l'une quelconque des revendications 1 à 3, caractérisé en ce que les moyens assurant l'étanchéité entre le boisseau (7) et chaque flasque (5,6) sont constitués par un système de joints solidaires du flasque ou du boisseau et engagés dans des rainures ménagées dans l'une ou l'autre de ces pièces, comprenant un joint annulaire (19) entourant l'axe de rotation, un joint annulaire (20) disposé à proximité de la périphérie des pièces et des joints (22) entourant les perçages ménagés axialement dans le boisseau.

5.- Robinet selon l'une quelconque des revendications 1 à 4, caractérisé en ce qu'il comporte, ménagée dans un flasque (5) ou dans la face correspondante du boisseau (7), une rainure circulaire (17) centrée sur l'axe de rotation (13) du boisseau (7) et située à même distance de l'axe que les perçages du boisseau, s'étendant à partir de l'un des perçages du flasque ou du boisseau et susceptible de mettre en communication la tubulure débouchant dans l'un des flasques avec le perçage du boisseau dans lequel débouche une tubulure radiale (16).

6.- Robinet selon la revendication 5, caractérisé en ce que le flasque (6) situé du côté opposé à celui (5) présentant une rainure de mise en communication d'une tubulure axiale et d'une tubulure radiale comporte une rainure circulaire (18) centrée sur l'axe de rotation (13) du boisseau et située à même distance de l'axe que les perçages du boisseau, s'étendant à partir de la tubulure du flasque dans lequel elle est ménagée, sur un angle correspondant au décalage angulaire des deux perçages ménagés dans le boisseau.

7.- Robinet selon l'une quelconque des revendications 1 à 6, caractérisé en ce qu'il est monté à l'intérieur d'un boîtier (24) comprenant une partie rigide contenant de la mousse (25) susceptible d'être imbibée d'agent antiseptique, le boîtier comprenant une fente disposée dans le plan du boisseau (7), destinée à laisser le passage des moyens d'actionnement de celui-ci.

8.- Robinet selon la revendication 7, caractérisé en ce que les moyens d'actionnement du boisseau sont constitués par un levier (23) radial, de longueur supérieure à la dimension radiale maximale du boîtier (24), considérée à partir de l'axe de rotation.

9.- Robinet selon l'une quelconque des revendications 7 et 8, caractérisé en ce que le boîtier (24) présente une dissymétrie de révolution, certaines zones se trouvant à une distance de l'axe de pivotement (13) inférieure à la distance entre la connexion de la tubulure radiale (16) et l'axe de rotation.

10.- Robinet selon la revendication 1, caractérisé en ce que les moyens d'actionnement du boisseau (7) sont constitués par un levier radial (30) solidaire de celui-ci et sortant du boîtier, sur lequel sont montés de façon pivotante élastiquement deux bras (32) qui en sont parallèles et qui sont disposés de part et d'autre de lui, une des extrémités libres de chacun de ces deux bras étant au contact d'une rampe fixe crantée (34, 35), lorsqu'aucune action n'est exercée sur ceux-ci, les crans (34) de chaque rampe (35) correspondant aux différentes positions de fonctionnement du boisseau.

FIG 1

FIG.2

FIG.3

6

13

18

12

30

7

19

20

8   9

5

22

23

16

15

14

10

FIG.4

23

7

6

5

12

10

3

25

24

FIG.5

28

29

4

8

30

19

9

12

20

7

5

10

6

16

FIG_6

FIG_7

FIG_8

FIG_9

FIG.10

FIG.11

## DOCUMENTS CONSIDERES COMME PERTINENTS

| Catégorie | Citation du document avec indication, en cas de besoin, des parties pertinentes | Revendication concernee | CLASSEMENT DE LA DEMANDE (Int. Cl.4) |
|---|---|---|---|
| A | EP-A-0 037 746 (MILBERGER)<br>* Figures 3,6 *<br>--- | 1 | F 16 K 11/074<br>A 61 M 5/16 |
| A | DE-A-2 160 739 (STEPHENS)<br>* Page 5, ligne 17 - page 7, ligne 24 *<br>--- | 1 | |
| A | US-A-3 713 462 (BUSHEE)<br>* Figure 14 *<br>--- | 1 | |
| A | FR-A-1 075 683 (ELECTRAULIC PRESSES)<br>* En entier *<br>--- | 1 | |
| A | FR-A-1 340 205 (SFERMA)<br>* Figure 1 *<br>----- | 1 | |

DOMAINES TECHNIQUES RECHERCHES (Int. Cl.4)

F 16 K
A 61 M

Le présent rapport a été établi pour toutes les revendications

| Lieu de la recherche | Date d'achèvement de la recherche | Examinateur |
|---|---|---|
| LA HAYE | 10-07-1989 | VERELST P.E.J. |